# EUROPÄISCHE PATENTANMELDUNG

(11) **EP 4 848 297 A1**
(43) Veröffentlichungstag der Anmeldung: **02.12.2026**
(21) Anmeldenummer: 26180484.3
(22) Anmeldetag: 22.05.2026
(51) Int. Cl.: G16H 20/17, G16H 40/40, G16H 40/67

(54) **MEDIZINISCHES SYSTEM, DATENÜBERTRAGUNGSSYSTEM UND VERFAHREN ZUM ABSICHERN VON EMPFANGENEN DATEN**

(30) Priorität: 28.05.2025 DE 102025120867
(71) Anmelder: B. Braun Avitum AG, 34212 Melsungen (DE)
(72) Erfinder: RIESS, Armin, 34587 Felsberg (DE); WESTPHAL, Martin, 34131 Kassel (DE)
(74) Vertreter: Winter, Brandl - Partnerschaft mbB

(57) **Zusammenfassung**

Offenbart ist ein medizinisches System (6), das aufweist: ein medizinisches Gerät (8) und eine Datenverarbeitungsvorrichtung (10), die zumindest teilweise in dem medizinischen Gerät (8) integriert ist und/oder mit diesem verbunden oder verbindbar ist. Die Datenverarbeitungsvorrichtung (10) ist konfiguriert, um Daten von einer externen Datenquelle zu empfangen oder mit dieser auszutauschen, empfangene Daten zu analysieren und die Daten nur zu verarbeiten, wenn sie einer oder mehreren Vorgaben entsprechen. Die Datenverarbeitungsvorrichtung (10) ist offenbarungsgemäß konfiguriert, um einen aktuellen Betriebszustand des medizinischen Geräts (8) zu überwachen und von der externen Datenquelle empfangene Daten nur weiter zu analysieren oder verarbeiten, wenn ein Empfang von Daten im aktuellen Betriebszustand vorgesehen ist und/oder Daten in Antwort auf eine von der Datenverarbeitungsvorrichtung (10) gestellte Anfrage empfangen werden.

## Beschreibung

Die vorliegende Offenbarung betrifft ein medizinisches System, das ein medizinisches Gerät, wie beispielsweise eine Infusionspumpe oder eine Dialysevorrichtung, und eine Datenverarbeitungsvorrichtung, die zumindest teilweise in dem medizinischen Gerät integriert ist und/oder mit diesem verbunden oder verbindbar ist, aufweist. Die Datenverarbeitungsvorrichtung ist konfiguriert, um Daten von einer externen Datenquelle, wie etwa von einem Server, zu empfangen oder mit dieser auszutauschen. Die empfangenen Daten werden durch die Datenverarbeitungsvorrichtung jedoch nur analysiert oder verarbeitet, wenn ein Empfang von Daten im aktuellen Betriebszustand des medizinischen Geräts vorgesehen ist und/oder Daten in Antwort auf eine von der Datenverarbeitungsvorrichtung gestellte Anfrage empfangen werden. Weiterhin betrifft die vorliegende Offenbarung ein Datenübertragungssystem, das die Datenquelle und mindestens ein medizinisches System aufweist. Außerdem betrifft die vorliegende Offenbarung ein Verfahren zum Absichern von empfangenen Daten, die von der externen Datenquelle bereitgestellt werden.

Es ist in der Medizintechnik bekannt, dass ein Server und mindestens ein medizinisches Gerät über eine authentifizierte und verschlüsselte Datenverbindung miteinander Daten austauschen.

Aus DE 10 2021 127 476 A1 ist ein System mit einer zentralen Medikamentendatenbank und zumindest einem externen, dezentralen medizinischen Gerät bekannt. Das System ist dazu vorgesehen und ausgebildet, eine Medikamentenverschreibung zu empfangen und einen entsprechenden Medikamentendatensatz für das zumindest eine medizinische Gerät abzurufen. Die zentrale Medikamentendatenbank weist eine Vielzahl an Medikamentendatensätzen für das zumindest eine medizinische Gerät auf und ist dazu vorgesehenen und ausgebildet, um die Medikamentenverschreibung mit der Vielzahl an Medikamentendatensätzen abzugleichen, zu validieren und vorzubereiten, um bei Verwendung des zumindest einen medizinischen Geräts nur den der Medikamentenverschreibung entsprechenden Medikamentendatensatz von der zentralen Medikamentendatenbank in das zumindest eine medizinische Gerät zu implementieren. Dabei wird bei Empfang des Medikamentendatensatzes von einem validen Datenstrom ausgegangen, bei dem bedeutungsrichte Daten zugespielt werden.

Angreifer / Hacker haben jedoch ein zunehmendes Interesse, Schad- oder Spionagesoftware in medizinische Netzwerke, wie etwa Krankenhaus- oder Kliniknetzwerke, einzuschleusen, um Daten, wie beispielsweise Betriebsdaten von medizinischen Geräten, abzugreifen oder anderweitig Schaden anzurichten.

Es ist Aufgabe der vorliegenden Offenbarung, eine Technik bereitzustellen, die ein schädliches Einschleusen von Schad- oder Spionagesoftware in ein medizinisches Netzwerk verhindert oder zumindest erschwert.

Diese Aufgabe wird durch die Gegenstände mit den Merkmalen gemäß den unabhängigen Ansprüchen gelöst. Vorteilhafte Ausführungsformen sind Gegenstand der abhängigen Ansprüche.

Ein offenbarungsgemäßes medizinisches System weist ein medizinisches Gerät auf. Das medizinische Gerät kann beispielsweise eine Infusionspumpe oder eine Dialysevorrichtung sein.

Das medizinische System weist weiter eine Datenverarbeitungsvorrichtung auf, die zumindest teilweise in dem medizinischen Gerät integriert ist und/oder mit diesem verbunden oder verbindbar ist. Die Datenverarbeitungsvorrichtung kann dabei auch vollständig in dem medizinischen Gerät integriert sein. Die Datenverarbeitungsvorrichtung kann beispielsweise durch mehrere Hardware-Komponenten gebildet sein und zumindest eine der Hardware-Komponenten kann in dem medizinischen Gerät integriert sein, während mindestens eine weitere Hardware-Komponente über eine geeignete Datenverbindung mit dem medizinischen Gerät, d.h. mit der in dem medizinischen Gerät integrierten Hardware-Komponente, verbunden ist. Es ist aber auch denkbar, dass die Datenverarbeitungsvorrichtung vollständig außerhalb des medizinischen Geräts angeordnet ist.

Die Datenverarbeitungsvorrichtung ist konfiguriert, um Daten von einer externen Datenquelle zu empfangen oder mit dieser auszutauschen. Die externe Datenquelle kann ein Server sein. Der Server kann ein dedizierter Server, d.h. ein physischer Server für einen bestimmten Anwendungsfall, ein virtueller Server, d.h. eine virtuelle Maschine auf einem physischen Server, oder ein Cloud-Server sein. Die externe Datenquelle kann auch ein externer Speicher, wie etwa ein Network Attached Storage (NAS), sein und die Datenverarbeitungsvorrichtung kann die Daten aus dem externen Speicher laden. Weiterhin ist es auch denkbar, dass die externe Datenquelle ein weiteres medizinisches System ist, das Daten an die Datenverarbeitungsvorrichtung des medizinischen Systems überträgt.

Die Daten können der Datenverarbeitungsvorrichtung bevorzugt über eine Netzwerkverbindung bereitgestellt bzw. an diese übertragen werden. Die Datenverarbeitungsvorrichtung kann hierzu eine geeignete Netzwerkschnittstelle zum Verbinden mit der Netzwerkverbindung aufweisen. Die Netzwerkverbindung kann ein Local Area Network, wie etwa ein Krankenhaus- oder Kliniknetzwerk, sein. Die Netzwerkverbindung kann auch ein Wide Area Network (WAN), wie etwa das Internet, sein. In Fall eines WAN kann eine Kommunikation der Datenverarbeitungsvorrichtung mit dem Server über eine gesicherte Verbindung, wie beispielsweise eine Virtual Private Network (VPN)- oder Transport-Layer-Security (TLS)-Verbindung, erfolgen.

Die Datenverarbeitungsvorrichtung ist konfiguriert, um einen aktuellen Betriebszustand des medizinischen Geräts zu überwachen bzw. zu erlangen. Hierzu kann beispielsweise ein Zustandsmodell in der Datenverarbeitungsvorrichtung implementiert sein. Die Datenverarbeitungsvorrichtung ist konfiguriert, um die empfangenen Daten nur zu analysieren oder zu verarbeiten, wenn ein Empfang von Daten im aktuellen Betriebszustand vorgesehen ist und/oder Daten in Antwort auf eine von der Datenverarbeitungsvorrichtung gestellte Anfrage empfangen werden. Unter Analysieren wird eine weitergehende Prüfung der Daten verstanden. Unter Verarbeiten wird dabei ein Steuern oder Betreiben des medizinischen Geräts entsprechend den empfangenen Daten verstanden. Die Datenverarbeitungsvorrichtung prüft also, ob im aktuellen Betriebszustand überhaupt neue, eingehende Daten erwartet werden. Wenn die Datenverarbeitungsvorrichtung eine Nachricht bzw. eine Antwort auf eine vorhergehende Anfrage erwartet, wie etwa am Anfang oder am Ende, nicht aber während einer Behandlung, werden eingehende Daten erst tiefgreifender analysiert oder verarbeitet. Demzufolge können möglicherweise schädliche Daten bereits zu einem frühen Zeitpunkt ohne weiterführende Analyse/Verarbeitung verworfen werden.

Gemäß einem Aspekt kann die Datenverarbeitungsvorrichtung konfiguriert sein, um die Daten syntaktisch zu analysieren und die Daten nur weiter zu analysieren oder zu verarbeiten, wenn sie einer syntaktischen Vorgabe entsprechen. Das heißt, die Datenverarbeitungsvorrichtung kann eine syntaktische Analyse bzw. Prüfung der empfangenen Daten durchführen und dabei prüfen, ob die in den Daten enthaltenen Datentypen überhaupt darin enthalten sein dürfen. Beispiele für Datentypen sind Integer und Textzeichen bzw. Character. Die Datenverarbeitungsvorrichtung prüft auch, ob ein erhaltener Datenpunkt innerhalb eines gültigen Datenbereichs liegt. Ein vorzeichenloser 16-Bit-Integer muss beispielsweise zwischen 0 und 65.564 liegen. Ein vorzeichenbehafteter 32 Bit Integer als bevorzugte Ausgestaltung muss beispielsweise zwischen -2.147.483.648 und 2.147.483.647 liegen. Dabei kann die Datenverarbeitungsvorrichtung konfiguriert sein, nur syntaktisch korrekte Daten werden weiter zu analysieren oder ggf. gleich zu verarbeiten. Andernfalls kann die Datenverarbeitungsvorrichtung konfiguriert sein, die syntaktisch fehlerhaften Daten zu verwerfen und keine weiterführende Analyse oder Verarbeitung der Daten durchzuführen. Durch die Durchführung der syntaktischen Prüfung sind ungültige Steuerzeichen in den Daten erkennbar, so dass eine SQL-Injection oder die Ausführung von angehängtem Binärcode durch Buffer Overflow wirksam verhindert wird.

Gemäß einem Aspekt kann die Datenverarbeitungsvorrichtung konfiguriert ist, um die Daten semantisch zu analysieren und die Daten nur zu verarbeiten, wenn sie einer semantischen Vorgabe entsprechen. Dabei kann eine semantische Analyse erst erfolgen, nachdem die Datenverarbeitungsvorrichtung festgestellt hat, dass die Daten der syntaktischen Vorgabe entsprechen. Das heißt, die Datenverarbeitungsvorrichtung kann konfiguriert sein, um vor Durchführung der semantischen Analyse die Daten syntaktisch zu analysieren. Bei einer semantischen Analyse werden Daten inhaltlich und kontextbezogen interpretiert, um deren Bedeutung (Semantik) zu erfassen. Ziel ist es, Informationen aus den Daten zu extrahieren, indem Zusammenhänge, Entitäten, Relationen, Bedeutungen und Kontexte erkannt und nutzbar gemacht werden. Weiterhin kann die Datenverarbeitungsvorrichtung konfiguriert sein, die Daten nur zu verarbeiten, wenn sie einer semantischen Vorgabe entsprechen, d.h. semantisch korrekt sind. Andernfalls ist die Datenverarbeitungsvorrichtung konfiguriert, um die Daten zu verwerfen und nicht weiter zu verarbeiten. Beispielsweise werden die Daten dann nicht für einen Betrieb des medizinischen Geräts herangezogen.

Gemäß einem weiteren Aspekt kann die Datenverarbeitungsvorrichtung konfiguriert sein, um die Daten zu typisieren, d.h. ein Typisieren der Daten durchzuführen. Die Datenverarbeitungsvorrichtung kann das Typisieren durchführen, bevor sie die Daten syntaktisch analysiert. Demzufolge werden nicht nur die in den Daten enthaltenen Nutzwerte, sondern auch der Protokollrahmen der erhaltenen Daten geprüft.

Gemäß einem zusätzlichen Aspekt kann die Datenverarbeitungsvorrichtung konfiguriert sein, um beim Typisieren der Daten ein Datenformat der Daten zu analysieren. Die Datenverarbeitungsvorrichtung kann dabei so konfiguriert sein, dass sie die Daten nur syntaktisch analysiert, wenn sie einem vorgegebenen Datenformat entsprechen. Demnach werden Daten, die in einem vorgegebenen Datenformat übertragen werden, überhaupt syntaktisch geprüft. Andernfalls ist die Datenverarbeitungsvorrichtung konfiguriert, um die Daten, die nicht dem vorgegebenen Datenformat entsprechen, zu verwerfen.

Das vorgegebene Datenformat kann dabei beispielsweise JavaScript Object Notation (JSON) oder Extensible Markup Language (XML) sein. Das Datenformat kann aber auch ein anderes Format, beispielsweise ein proprietäres Datenformat, sein.

Gemäß einem weiteren Aspekt kann die Datenverarbeitungsvorrichtung konfiguriert sein, um beim Typisieren der Daten einen Dateninhalt der Daten zu analysieren. Die Datenverarbeitungsvorrichtung kann dabei so konfiguriert sein, dass sie die Daten nur syntaktisch analysiert, wenn sie einem vorgegebenen Dateninhalt entsprechen. Der vorgegebene Dateninhalt bzw. eine definierte Typisierung werden dabei vorhergehend in der Datenverarbeitungsvorrichtung implementiert. Falls Daten erhalten werden, die nicht dem vorgegebenen Dateninhalt entsprechen, werden diese verworfen und nicht weiter syntaktisch analysiert.

Der vorgegebene Dateninhalt kann beispielsweise ein Medikamentendatensatz für das medizinische Gerät, eine Statusnachricht, eine Gerätekonfiguration, ein Software-Update und/oder ein Firmware-Update sein.

Gemäß einer Implementierung kann die Datenverarbeitungsvorrichtung eine Kommunikationseinheit und eine Betriebssteuereinheit aufweisen. Die Kommunikationseinheit und die Betriebssteuereinheit können physikalisch getrennt als separate Hardware-Komponenten ausgebildet sein. Die separaten Hardware-Komponenten können dabei auch weitere Aufgaben, wie etwa als Firewall, ausführen. Weiterhin ist es auch denkbar, dass die Kommunikationseinheit und die Betriebssteuereinheit logisch getrennt in einer gemeinsamen Hardware-Einheit implementiert sind. Die Kommunikationseinheit und die Betriebssteuereinheit sind dabei für einen bidirektionalen Datenaustausch miteinander konfiguriert. Die Kommunikationseinheit kann konfiguriert sein, um Daten von der Datenquelle zu empfangen oder Daten mit der Datenquelle auszutauschen, also mit der Datenquelle bidirektional zu kommunizieren. Die Betriebssteuereinheit kann konfiguriert sein, um einen Betrieb des medizinischen Geräts zu steuern.

Weiterhin kann die Kommunikationseinheit konfiguriert sein, um zu prüfen, ob ein Empfang von Daten im aktuellen Betriebszustand vorgesehen ist und/oder Daten in Antwort auf eine Anfrage empfangen wurden. Die Kommunikationseinheit kann hierfür konfiguriert sein, um den aktuellen Betriebszustand von der Betriebssteuereinheit zu erlangen bzw. abzufragen. Zum Ermitteln des Betriebszustands kann in der Betriebssteuereinheit ein Zustandsmodell implementiert sein. Alternativ oder zusätzlich kann die Kommunikationseinheit konfiguriert sein, um die Daten zu typisieren. Alternativ oder zusätzlich kann die Kommunikationseinheit konfiguriert sein, um die Daten syntaktisch zu analysieren. Die Betriebssteuereinheit kann konfiguriert sein, um die Daten semantisch zu analysieren. Demnach kann die Kommunikationseinheit in medizinischen Geräten, die bereits eine Betriebssteuereinheit aufweisen, nachgerüstet werden, um zumindest eine syntaktische Analyse der eingehenden Daten durchzuführen.

Gemäß einer Implementierung können die Kommunikationseinheit und die Betriebssteuereinheit in dem medizinischen Gerät integriert sein. Wie bereits erwähnt, können die Kommunikationseinheit und die Betriebssteuereinheit logisch oder physikalisch getrennt implementiert sein. Demzufolge kann eine Nachrüstung der Kommunikationseinheit, die die syntaktische Prüfung ermöglicht, auf einfache Weise erfolgen.

Gemäß einer anderen Implementierung kann die Betriebssteuereinheit in dem medizinischen Gerät integriert sein. Die Kommunikationseinheit kann als ein zu dem medizinischen Gerät externes Gerät ausgebildet sein und mit dem medizinischen Gerät, d.h. mit der Betriebssteuereinheit, verbunden oder verbindbar sein. In diesem Fall kann die Kommunikationseinheit als separate Hardware-Komponente ausgebildet sein. Alternativ kann die Kommunikationseinheit in einem zu dem medizinischen Gerät externen Gerät integriert sein. Das heißt, das externe Gerät, wie etwa ein Switch, ein Gateway oder ein Router, kann bereits vorhanden sein und die Kommunikationseinheit kann darin logisch oder auch physikalisch implementiert und somit auf einfache Weise nachgerüstet werden.

Gemäß einem zusätzlichen Aspekt kann die Kommunikationseinheit konfiguriert sein, um mit mehreren Betriebssteuereinheiten von mehreren medizinischen Geräten verbunden oder verbindbar zu sein. Demzufolge kann die Kommunikationseinheit als ein Switch, ein Gateway oder ein Router ausgeführt sein. Demzufolge kann die Kommunikationseinheit für mehrere medizinische Geräte auf einfache Weise nachgerüstet werden.

Ein offenbarungsgemäßes Datenübertragungssystem weist eine Datenquelle, die konfiguriert ist, um mindestens einer Datenverarbeitungsvorrichtung eines medizinischen Geräts Daten bereitzustellen, auf. Die Datenquelle kann entsprechend den obigen Ausführungen ausgebildet sein. Die Daten können bevorzugt über eine oben beschriebene Netzwerkverbindung übermittelt werden. Weiterhin weist das Datenübertragungssystem mindestens ein medizinisches System gemäß einem der vorhergehenden Aspekte auf. Das medizinische System kann dabei gemäß den oben beschriebenen Aspekten weitergebildet werden. Demzufolge können durch das Datenübertragungssystem die oben beschriebenen Effekte und Wirkungen erreicht werden.

Weiterhin ist ein Verfahren zum Absichern von empfangen Daten, die von einer externen Datenquelle, insbesondere einem Server und insbesondere über eine Netzwerkverbindung, bereitgestellt werden, offenbart. Zunächst werden die von der externen Datenquelle bereitgestellten Daten durch die Datenverarbeitungsvorrichtung, die zumindest teilweise in dem medizinischen Gerät integriert ist und/oder mit diesem verbunden oder verbindbar ist, empfangen. Zudem erfolgt ein Überwachen des aktuellen Betriebszustands des medizinischen Geräts durch die Datenverarbeitungsvorrichtung. Außerdem erfolgt ein Analysieren oder Verarbeiten der von der externen Datenquelle empfangenen Daten durch die Datenverarbeitungsvorrichtung, wenn ein Empfang von Daten im aktuellen Betriebszustand vorgesehen ist und/oder Daten in Antwort auf eine von der Datenverarbeitungsvorrichtung gestellte Anfrage empfangen werden. Demzufolge können möglicherweise schädliche Daten bereits zu einem frühen Zeitpunkt ohne weiterführende Analyse/Verarbeitung verworfen werden.

Ein beispielhafter Ablauf des offenbarten Verfahrens kann dabei die nachfolgenden Schritte aufweisen, wobei einzelne Schritte auch weggelassen werden können:
Anfragen von Daten durch die Datenverarbeitungsvorrichtung des medizinischen Geräts;
Übertragen der angefragten Daten durch die Datenquelle, insbesondere den Server, an die anfragende Datenverarbeitungsvorrichtung;
Empfangen der Daten durch die Datenverarbeitungsvorrichtung;
Prüfen, ob Daten angefragt wurden und/oder ein Empfang von Daten im gegenwärtigen Betriebszustand vorgesehen ist;
wenn Daten angefragt wurden und/oder ein Empfang von Daten im gegenwärtigen Betriebszustand vorgesehen ist, Typisieren der Daten;
wobei das Typisieren der Daten ein Prüfen eines Datenformats der Daten und/oder eines Dateninhaltes der Daten umfasst,
wenn die Daten eine korrekte Typisierung aufweisen, syntaktische Analyse der Daten;
wenn die Daten syntaktisch korrekt sind, semantische Analyse der Daten;
wenn die Daten semantisch korrekt sind, Verarbeiten der Daten, um beispielsweise das medizinische Gerät entsprechend zu betreiben.

Nachfolgend werden Ausführungsformen der vorliegenden Offenbarung unter Bezugnahme auf die Figuren beschrieben. Es zeigen:
Fig. 1 eine schematische Ansicht eines Datenübertragungssystems gemäß einer Ausführungsform;
Fig. 2 ein schematisches Blockschaltbild eines medizinischen Geräts gemäß einer Ausführungsform mit einer integrierten Datenverarbeitungsvorrichtung;
Fig. 3 eine schematische Ansicht eines Datenübertragungssystems gemäß einer weiteren Ausführungsform; und
Fig. 4 ein beispielhaftes Ablaufdiagramm eines Verfahrens zum Absichern von empfangenen Daten gemäß einer Ausführungsform.

Nachfolgend werden Ausführungsformen der vorliegenden Offenbarung unter Bezugnahme auf die Figuren beschrieben. Diese nachfolgende Beschreibung der Ausführungsformen ist jedoch nicht dahingehend auszulegen, dass der Schutzumfang der folgenden Offenbarung in irgendeiner Weise dadurch begrenzt werden soll.

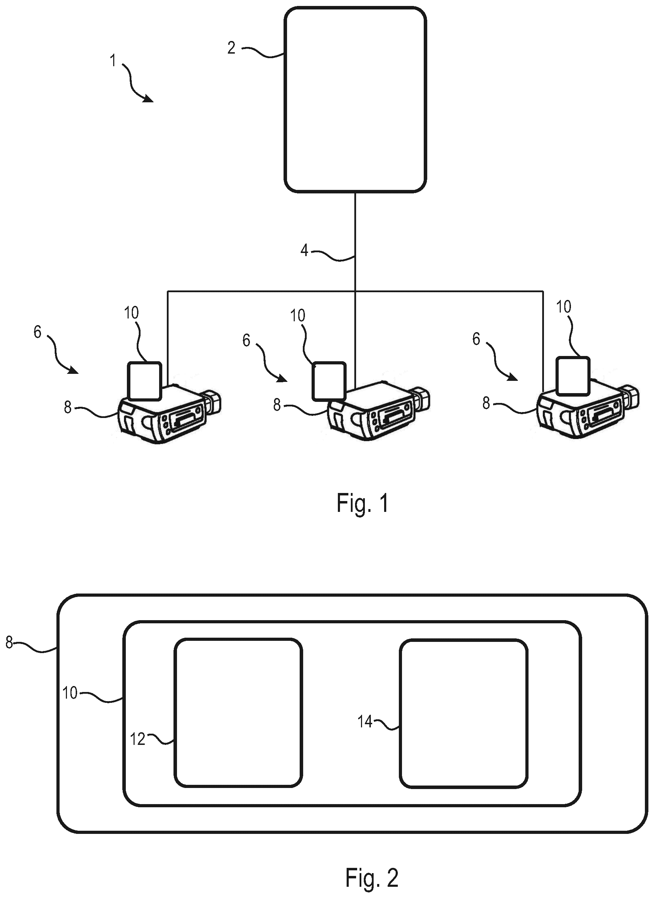

Fig. 1 zeigt eine schematische Ansicht eines Datenübertragungssystems 1 gemäß einer Ausführungsform, das einen Server 2 als ein Beispiel einer Datenquelle, eine Netzwerkverbindung 4 und drei medizinische Systeme 6 aufweist. Die medizinischen Systeme 6 weisen jeweils ein medizinisches Gerät 8 und eine Datenverarbeitungsvorrichtung 10 auf. Es ist anzumerken, dass die Anzahl der medizinischen Systeme 6 nicht auf die gezeigte beispielshafte Anzahl von drei begrenzt ist.

Der Server 2 ist in der vorliegenden Ausführungsform beispielhaft als ein zentraler Server-Rechner ausgebildet. Die Netzwerkverbindung 4 kann dann beispielhaft eine lokale Netzwerkverbindung, wie etwa ein Klinik- oder Krankenhausnetzwerk, sein.

Als ein Beispiel für die medizinischen Geräte 8 der medizinischen Systeme 6 sind in Fig. 1 Infusionspumpen gezeigt. Die Datenverarbeitungsvorrichtungen 10 der medizinischen Geräte 8 können in diesem Fall kabelgebunden oder kabellos mit der Netzwerkverbindung 4 verbunden sein. Es ist aber auch die Verwendung anderer Netzwerkarchitekturen und medizinischer Geräte 8, wie sie beispielsweise eingangs der vorliegenden Patentanmeldung beschrieben sind, denkbar.

Der Server 2 ist konfiguriert, um mit der in Fig. 2 dargestellten Datenverarbeitungsvorrichtung 10 des medizinischen Systems 6 bidirektional zu kommunizieren, also Daten in beide Richtungen auszutauschen. Die Datenverarbeitungsvorrichtung 10 ist in der vorliegenden Ausführungsform, wie in Fig. 2 gezeigt, in dem medizinischen Gerät 8 vollständig integriert und weist eine Kommunikationseinheit 12 und eine Betriebssteuereinheit 14 auf. Dabei können die Kommunikationseinheit 12 und die Betriebssteuereinheit 14 als separate Hardware-Einheiten ausgebildet sein. Die Kommunikationseinheit 12 ist konfiguriert, um mit dem Server 2 zu kommunizieren, und die Betriebssteuereinheit 14 ist konfiguriert, um einen Betrieb des medizinischen Geräts 8 zu steuern. Demzufolge ist es möglich, die durch die Kommunikationseinheit 12 ausführbaren Funktionen auch in bestehende medizinische Gerate, die lediglich die Betriebssteuereinheit 14 aufweisen, nachzurüsten. Die Ausbildung der Steuereinheit 10 als zwei separate Hardware-Einheiten ist beispielhaft und es ist ebenso denkbar, dass die Kommunikationseinheit 12 und die Betriebssteuereinheit 14 innerhalb einer Hardware-Einheit als logisch getrennte Einheiten ausgebildet sind.

Die Kommunikationseinheit 12 überwacht kontinuierlich einen aktuellen Betriebszustand des medizinischen Geräts 8, indem sie den aktuellen Betriebszustand von der Betriebssteuereinheit 14 kontinuierlich erlangt. In der Betriebssteuereinheit 14 kann zum Feststellen des aktuellen Betriebszustands ein Zustandsmodell implementiert sein. Werden Daten von dem Server 2 empfangen, dann werden diese nur weiter analysiert, wenn ein Empfang von Daten im aktuellen Betriebszustand überhaupt vorgesehen ist. Alternativ oder zusätzlich kann die Kommunikationseinheit 12 so ausgebildet sein, dass sie Daten nur weiter analysiert, wenn diese in Antwort auf eine vorhergehend an den Server 2 gestellte Anfrage empfangen werden. Beispielsweise stellt die Kommunikationseinheit 12 fest, dass eine Behandlung mit dem medizinischen Gerät 8 noch nicht gestartet ist und eine Medikamentenverschreibung eingegeben wurde. Die Medikamentenverschreibung wird anschließend auf dem Server 2 mit vorhandenen Medikamentendatensätzen abglichen und validiert. Ein der Medikamentenverschreibung entsprechender Medikamentendatensatz wird dann von dem Server 2 an die Kommunikationseinheit 12 übertragen. Da in dem aktuellen Betriebszustand mit einem Empfang des Medikamentendatensatzes zu rechnen ist und dieser in Antwort auf eine vorher übertragene Medikamentenverschreibung erhalten wird, bestimmt die Kommunikationseinheit 12, dass die empfangenen Daten weiter zu analysieren sind. Andernfalls würde die Kommunikationseinheit 12 die empfangenen Daten verwerfen und nicht weiter analysieren oder gar verarbeiten.

Weiterhin ist die Kommunikationseinheit 12 konfiguriert, um die empfangenen Daten zu typisieren. Dabei kann die Kommunikationseinheit 12 konfiguriert sein, um beim Typisieren der Daten ein Datenformat der Daten zu analysieren und die Daten nur weiter zu analysieren, wenn sie einem vorgegebenen Datenformat entsprechen. Das vorgegebene Datenformat kann beispielsweise JavaScript Object Notation (JSON) oder Extensible Markup Language (XML) sein. Die Verwendung anderer Datenformate, wie etwa proprietärer Datenformate, ist aber ebenso denkbar.

Alternativ oder zusätzlich kann die Kommunikationseinheit 12 konfiguriert sein, um beim Typisieren der Daten einen Dateninhalt der Daten zu analysieren und die Daten nur weiter zu analysieren, wenn sie einem vorgegebenen Dateninhalt entsprechen. Beispielsweise kann der vorgegebene Dateninhalt ein Medikamentendatensatz sein, der nachfolgend zu einer Übertragung einer Medikamentenverschreibung von dem Server 2 erhalten wird. Weiterhin kann der Dateninhalt eine Statusnachricht, eine Gerätekonfiguration, ein Software-Update und/oder ein Firmware-Update sein.

Die Kommunikationseinheit 12 kann weiter konfiguriert sein, um anschließend die Daten syntaktisch zu analysieren und die Daten nur weiter zu analysieren, wenn sie einer syntaktischen Vorgabe entsprechen, also syntaktisch korrekt sind. Durch die Durchführung der syntaktischen Analyse kann verhindert werden, dass dem medizinischen Gerät 8 ungültige Daten zugespielt werden und es außer Takt kommt, da es versucht den erhaltenen Wert als Zahl zu lesen, was er jedoch nicht ist. Demzufolge kann ein typisches Angriffsmuster, wie etwa eine SQL-Injection Schwachstelle, verhindert werden.

Weiterhin kann die Betriebssteuereinheit 14 konfiguriert sein, um die Daten semantisch zu analysieren und die Daten nur zu verarbeiten, wenn sie einer semantischen Vorgabe entsprechen, also semantisch korrekt sind. Demzufolge erfolgt der Betrieb des medizinischen Geräts 8 nur entsprechend Daten, die vorhergehend umfassend analysiert bzw. geprüft wurden, so dass das Einschleusen von Schad- oder Spionagesoftware in das medizinische Gerät 8 verhindert oder zumindest erschwert wird.

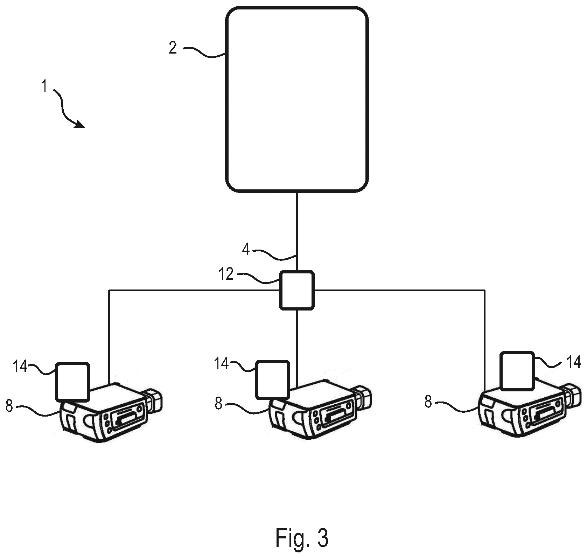

Fig. 3 zeigt eine schematische Ansicht eines Datenübertragungssystems 1 gemäß einer weiteren Ausführungsform, das einen Server 2 als ein Beispiel einer Datenquelle, eine Netzwerkverbindung 4 und drei medizinische Systeme 6 aufweist. Die in Fig. 3 gezeigte Ausführungsform unterscheidet sich von der in Fig. 1 gezeigten Ausführungsform dadurch, dass die Kommunikationseinheit 12 und die Betriebssteuereinheit 14 als separaten Hardware-Einheiten ausgebildet sind und nur die Betriebssteuereinheit 14 in dem medizinischen Gerät 8 integriert ist. Weiterhin ist die Kommunikationseinheit 12 mit den Betriebssteuereinheiten 14 der drei medizinischen Systeme 6 bzw. der drei medizinischen Geräte 8 verbunden. Die Funktionen der Kommunikationseinheit 12 und der Betriebssteuereinheit entsprechen dabei den oben beschriebenen Funktionen. Es ist aber ebenso denkbar, dass pro medizinischem Gerät 8, d.h. Betriebssteuereinheit 14, eine Kommunikationseinheit 12 vorgesehen ist. Es ist darüber hinaus denkbar, dass eine Kommunikationseinheit 12 mit einem medizinischen Gerät 8 verbunden ist und eine weitere Kommunikationseinheit 12 mit mehreren weiteren medizinischen Geräten 8 verbunden ist. Wiederum ist die Anzahl der medizinischen Systeme bzw. medizinischen Geräte 8 nicht auf die beispielshaft gezeigte Anzahl von drei begrenzt. Weiterhin ist auch eine Kombination der in Fig. 1 gezeigten Ausführungsform mit der in Fig. 3 gezeigten Ausführungsform möglich.

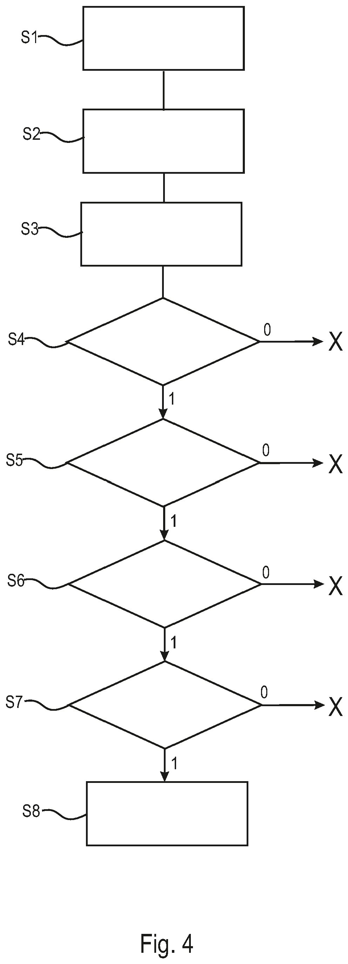

Nachfolgend wird ein beispielhafter Ablauf eines offenbarungsgemäßen Verfahrens anhand dem in Fig. 4 gezeigten Ablaufdiagramm beschrieben. Es ist anzumerken, dass der gezeigte Ablauf nur beispielhaft ist und einzelne Schritte auch weggelassen werden können.

In Schritt S1 überträgt die Datenverarbeitungsvorrichtung 10, d.h. die Kommunikationseinheit 12 auf Anweisung der Betriebssteuereinheit 14, eine Anfrage an den Server 2. Im nachfolgenden Schritt S2 werden Daten durch den Server 2 in Antwort auf die Anfrage an die Datenverarbeitungsvorrichtung 10 übertragen. In Schritt S3 werden die Daten durch die Datenverarbeitungsvorrichtung 10 empfangen. In Schritt S4 prüft die Datenverarbeitungsvorrichtung 10, z.B. die Kommunikationseinheit 12, ob die Daten angefragt wurden und/oder ein Empfang von Daten im gegenwärtigen Betriebszustand vorgesehen ist. Ist diese Prüfung negativ (0 in S4), werden die Daten, wie durch das X in Fig. 4 angedeutet, verworfen. Ist die Prüfung jedoch positiv (1 in S4), werden die Daten in S5 weiter analysiert. In Schritt S5 führt die Datenverarbeitungsvorrichtung 10, z.B. die Kommunikationseinheit 12, eine Typisierung der Daten durch. Bei der Typisierung der Daten können beispielsweise ein Datenformat und/oder ein Dateninhalt der erhaltenen Daten überprüft werden. Ist die Typisierung negativ (0 in S5), werden die Daten verworfen. Ist die Typisierung hingegen positiv (1 in S5), werden die Daten in Schritt S6 weiter analysiert. In Schritt S6 führt die Datenverarbeitungsvorrichtung 10, z.B. die Kommunikationseinheit 12, eine syntaktische Analyse der Daten durch. Sind die Daten syntaktisch fehlerhaft (0 in S6), werden die Daten verworfen. Andernfalls (1 in S6) werden die Daten in Schritt S7 weiter analysiert. In Schritt S7 führt die Datenverarbeitungsvorrichtung 10, z.B. die Betriebssteuereinheit 14, eine semantische Analyse der Daten durch. Ist diese semantische Analyse fehlerhaft (0 in S7), werden die Daten verworfen. Für den Fall, dass die Daten semantisch korrekt sind (1 in S7), werden die Daten in Schritt S8 durch die Datenverarbeitungsvorrichtung 10, z.B. die Betriebssteuereinheit 14, weiterverarbeitet, um beispielsweise eine Behandlung mit dem medizinischen Gerät 8 entsprechend den erhaltenen Daten durchzuführen.

### Bezugszeichenliste

- 1: Datenübertragungssystem
- 2: Server
- 4: Netzwerkverbindung
- 6: medizinisches System
- 8: medizinisches Gerät
- 10: Datenverarbeitungsvorrichtung
- 12: Kommunikationseinheit
- 14: Betriebssteuereinheit
- S1 bis S8: Verfahrensschritte

## Patentansprüche

1. Medizinisches System (6), das aufweist:
ein medizinisches Gerät (8), insbesondere eine Infusionspumpe oder eine Dialysevorrichtung, und
eine Datenverarbeitungsvorrichtung (10), die zumindest teilweise in dem medizinischen Gerät (8) integriert ist und/oder mit diesem verbunden oder verbindbar ist, wobei die Datenverarbeitungsvorrichtung (10) konfiguriert ist, um Daten von einer externen Datenquelle, insbesondere von einem Server (2) und insbesondere über eine Netzwerkverbindung (4), zu empfangen oder mit dieser auszutauschen,
**dadurch gekennzeichnet, dass**
die Datenverarbeitungsvorrichtung (10) konfiguriert ist, um einen aktuellen Betriebszustand des medizinischen Geräts (8) zu überwachen und von der externen Datenquelle empfangene Daten nur zu analysieren oder verarbeiten, wenn ein Empfang von Daten im aktuellen Betriebszustand vorgesehen ist und/oder Daten in Antwort auf eine von der Datenverarbeitungsvorrichtung (10) gestellte Anfrage empfangen werden.

2. Medizinisches System gemäß Anspruch 1, wobei
die Datenverarbeitungsvorrichtung (10) ferner konfiguriert ist, um die Daten syntaktisch zu analysieren und die Daten nur weiter zu analysieren oder zu verarbeiten, wenn sie einer syntaktischen Vorgabe entsprechen.

3. Medizinisches System (6) gemäß Anspruch 1 oder 2, wobei
die Datenverarbeitungsvorrichtung (10) konfiguriert ist, um die Daten semantisch zu analysieren und die Daten nur zu verarbeiten, wenn sie einer semantischen Vorgabe entsprechen.

4. Medizinisches System (6) gemäß einem der Ansprüche 1 bis 3, wobei
die Datenverarbeitungsvorrichtung (10) konfiguriert ist, um die Daten zu typisieren.

5. Medizinisches System (6) gemäß Anspruch 4, wobei
die Datenverarbeitungsvorrichtung (10) konfiguriert ist, um beim Typisieren der Daten ein Datenformat der Daten zu analysieren.

6. Medizinisches System (6) gemäß Anspruch 4 oder 5, wobei
die Datenverarbeitungsvorrichtung (10) konfiguriert ist, um beim Typisieren der Daten einen Dateninhalt der Daten zu analysieren.

7. Medizinisches System (6) gemäß Anspruch 6, wobei
der vorgegebene Dateninhalt ein Medikamentendatensatz, eine Statusnachricht, eine Gerätekonfiguration, ein Software-Update und/oder ein Firmware-Update ist.

8. Medizinisches System (6) nach einem der Ansprüche 1 bis 7, wobei
die Datenverarbeitungsvorrichtung (10) eine Kommunikationseinheit (12), die konfiguriert ist, um Daten von der externen Datenquelle, insbesondere von dem Server (2), zu empfangen, und eine Betriebssteuereinheit (14), die konfiguriert ist, um einen Betrieb des medizinischen Geräts (8) zu steuern, aufweist,
wobei die Kommunikationseinheit (10) konfiguriert ist, um zu prüfen, ob ein Empfang von Daten im aktuellen Betriebszustand vorgesehen ist und/oder Daten in Antwort auf eine Anfrage empfangen wurden, und/oder die Daten zu typisieren, und/oder die Daten syntaktisch zu analysieren, und die Betriebssteuereinheit (14) konfiguriert ist, um die Daten semantisch zu analysieren und/oder die Daten zu verarbeiten.

9. Medizinisches System (6) gemäß Anspruch 8, wobei
die Kommunikationseinheit (12) und die Betriebssteuereinheit (14) in dem medizinischen Gerät (8) integriert sind.

10. Medizinisches System (6) gemäß Anspruch 8, wobei
die Betriebssteuereinheit (14) in dem medizinischen Gerät (8) integriert ist, und
die Kommunikationseinheit (12) als ein zu dem medizinischen Gerät (8) externes Gerät ausgebildet oder in einem zu dem medizinischen Gerät (8) externen Gerät integriert ist.

11. Medizinisches System (6) gemäß Anspruch 10,
wobei die Kommunikationseinheit (10) konfiguriert ist, um mit mehreren Betriebssteuereinheiten (12) von mehreren medizinischen Geräten (6) verbunden oder verbindbar zu sein.

12. Datenübertragungssystem (1), das aufweist:
eine Datenquelle, insbesondere einen Server (2), die konfiguriert ist, um mindestens einer Datenverarbeitungsvorrichtung (10) eines medizinischen Geräts (8) Daten, insbesondere über eine Netzwerkverbindung (4), bereitzustellen, und
mindestens ein medizinisches System (6) gemäß einem der Ansprüche 1 bis 11.

13. Verfahren zum Absichern von empfangen Daten, die von einer externen Datenquelle, insbesondere einem Server (2) und insbesondere über eine Netzwerkverbindung (4), bereitgestellt werden, wobei das Verfahren die Schritte aufweist:
Empfangen der von der externen Datenquelle bereitgestellten Daten durch eine Datenverarbeitungsvorrichtung (10), die zumindest teilweise in einem medizinischen Gerät (8) integriert ist und/oder mit diesem verbunden oder verbindbar ist,
Überwachen des aktuellen Betriebszustands des medizinischen Geräts (8) durch die Datenverarbeitungsvorrichtung (10),
Analysieren oder Verarbeiten der von der externen Datenquelle empfangenen Daten durch die Datenverarbeitungsvorrichtung (10), wenn ein Empfang von Daten im aktuellen Betriebszustand vorgesehen ist und/oder Daten in Antwort auf eine von der Datenverarbeitungsvorrichtung (10) gestellte Anfrage empfangen werden.
